# EUROPEAN PATENT APPLICATION

(11) **EP 1 880 697 A1**
(43) Date of publication of application: **23.01.2008**
(21) Application number: 05824041.7
(22) Date of filing: 29.12.2005
(51) Int. Cl.: A61F 7/00

(54) **A SKIN CARE METHOD USING WAX AND THE APPARATUS THEREOF**

(30) Priority: 28.04.2005 CN 200510034484
(71) Applicant: Vincent Raya (Dong Guan) Electronics Co., Ltd., Dong Guan, Guangdong 523730 (CN)
(72) Inventor: CHOI, Vincent, Dong Guan,Guangdong 523730 (CN); HANG, Nigel, Dong Guan,Guangdong 523730 (CN); YANG, Zishen, Dong Guan,Guangdong 523730 (CN); HUANG, Sibin, Dong Guan,Guangdong 523730 (CN)
(74) Representative: Modin, Jan
(86) International application number: PCT/CN2005/002372
(87) International publication number: WO 2006/114033

(57) **Abstract**

A skin-treatment method using wax is provided, which includes preheating a skin-treatment wax filled in containers with nozzles in a heating device to melt the wax into a liquid wax capable of being sprayed, and spraying the liquid wax filled in the containers with nozzles in the heating device on a surface of the skin under treatment so as to form a wax mask. A skin-treatment tool using wax is also provided, which includes a heating device for preheating a skin-treatment wax placed therein to melt the wax into a liquid wax capable of being sprayed, and at least one container with a nozzle disposed in the heating device. The skin-treatment tool of the present invention has a compact structure and is convenient to use, which has been widely used in beauty parlors, houses, apartments, hotel rooms, lounges, and other places capable of conducting skin treatments using wax on the spot. The method and tool of the present invention do not generate cross infection of skin bacteria and virus, which meet the sanitary requirements. The liquid wax is not solidified during the spraying process away from the heat source, and the operation is continuously performed.

## Description

### Field of the Invention

The present invention relates to a skin protection method and a tool thereof, partially applied on the skin area without taking the skin care as an objective. More particularly, the present invention relates to a skin-treatment method using wax and a tool thereof.

### Background of the Invention

Paraffin is a high molecular hydrocarbon having features of a high heat capacity, small thermal conductivity, almost no heat convection, and desirable heat preservation. In the current skin-treatment method using wax, medical paraffin or treatment use formulated wax melted upon being heating is used as a warming medium, which generates a squeezing effect and a smoothing effect on the skin being covered by the paraffin during the cooling process, such that the heat energy stored in the paraffin is delivered to the deep tissues via the surface of the skin, so as to promote sweat glands to discharge sweats and metabolism effect of skin tissues, to expand the capillary vessel, to accelerate the blood circulation, to improve the skin tissue nutrition, to remove aging skin cells, to increase the permeability for the tissue cells, to relax the tightened skin tissue and nerves, and to ease fatigue. If the treatment use prepared wax is added by nutritive substances that are easily absorbed by the skin, for example, Vitamin E, collagen, and plant essential oils including lavender, peach, and tea, etc., it can be further used to prevent and modify desquamation and chapping problems of the skin, so as to make the skin smoother and tender, especially suitable for the treatment of dry, aging, and rough skins. However, the current skin-treatment method using wax mainly includes a paraffin brush method and a paraffin bath method. As for the former method, the paraffin is preheated to be melted to a liquid wax, then a brush or a blade is used to coat the liquid wax on the surface of the skin under treatment, the wax is repeatedly coated to form a wax mask on the surface of the skin, and then a plastic thin film or fabrics is used to wrap the skin for treatment. For the latter method, the paraffin is preheated to be melted into the liquid wax, a thin layer of wax is partially coated on the surface of the skin under treatment through the paraffin brush method, and then, the skin is quickly immerged in a bath filled with the liquid wax, and then taken out immediately, which is repeated for several times, so the wax mask is formed on the surface of the skin, and then the plastic thin film or fabric is used to wrap the skin for treatment. The brush or blade, and the bath, basin or bottle, and pot-shaped containers for holding the wax are repeatedly used for different people, so it is extremely easy to generate cross infection of the skin bacteria and virus, which do not meet the sanitary requirements. During the cooling process, it is extremely easy for the skin-treatment wax to be solidified, so the skin-treatment wax cannot be moved away from the heating device to perform the operation continuously through the paraffin brush method.

### SUMMARY OF THE INVENTION

The technical problem to be solved by the present invention is to overcome the disadvantages of the prior art, and to provide a skin-treatment method using wax that does not generate the cross infection of the skin bacteria and virus, and capable of being continuously operated away from the heat source.

Another technical problem to be solved by the present invention is to overcome the disadvantages of the prior art, and to provide a skin-treatment tool using wax that does not generate the cross infection of the skin bacteria and virus, and capable of being continuously operated away from the heat source.

Through the skin-treatment method using wax of the present invention, the above technical problem is solved as follows.

The skin-treatment method using wax sequentially includes the following steps: preheating a skin-treatment wax to melt the wax into a liquid wax, cleaning the skin under treatment, forming a wax mask on the surface of the skin under treatment, performing a sealed heat-insulation and heat-preservation process on the wax mask, and removing the solidified and thoroughly-dried wax mask.

The skin-treatment method using wax is characterized in that, the step of preheating the skin-treatment wax to melt the wax into the liquid wax is to preheat a skin-treatment wax filled in containers with nozzles in a heating device to melt the wax to a liquid wax capable of being sprayed; and the step of forming the wax mask on the surface of the skin under treatment is to spray the liquid wax filled in the containers with nozzles in the heating device onto the surface of the skin under treatment, so as to form the wax mask.

In this manner, it does not require to use the brush or blade to coat the liquid wax on the surface of the skin under treatment, or to immerge the surface of the skin under treatment within the bath filled with the liquid wax. Therefore, the cross infection of the skin bacteria and virus is not generated, and the liquid wax in the containers with nozzles is not solidified during the spraying process away from the heating device, and thus can be operated continuously.

As for the skin-treatment method using wax of the present invention, the above technical problem can be preferably solved as follows.

The step of spraying the liquid wax on the surface of the skin under treatment to form the wax mask is to uniformly spray the liquid wax to form a layer of wax and then to repeatedly spray the liquid wax to form several layers of wax, so as to form the wax mask.

The step of spraying the liquid wax on the surface of the skin under treatment to form the wax mask is to repeatedly spray the liquid wax to form at least two layers of wax, and the thickness of the wax mask formed on the surface of the skin under treatment reaches up to 1.2 mm-1.6 mm, such that the heat energy stored in the paraffin can be delivered to the deep tissues with the depth of about 1 cm via the surface of the skin, and thus, effective squeezing and smoothing effects are generated on the skin covered by the wax during the cooling process.

The heating device is a temperature control heating device, and the heating temperature range is 45°C to 85°C, so as to cater to the melting temperature characteristics of different kinds of skin-treatment waxes, and thus, the heated skin-treatment wax keeps the liquid state capable for being sprayed.

The liquid wax capable of being sprayed has a kinemetic viscosity cSt of 3.2 centistokes to 4.2 centistokes (98.9°C), or a Saybolt Universal Second (SUS) of 37 seconds to 40 seconds (98.9°C), so that it can be smoothly sprayed from the nozzle.

As for the skin-treatment method using wax of the present invention, the above technical problem can be further solved as follows.

The skin-treatment wax is paraffin, preferably medical paraffin, or treatment use formulated wax with nutritive substances easily absorbed by the skin added therein.

The paraffin (paraffin, paraffin wax or ceresin wax) is a solid hydrocarbon mixture, the molecular formula is CₙH₂ₙ₊₂, which is also called hard paraffin and paraffin hydrocarbon. The melting point of high-purity medical paraffin is 50°C-54°C, the oil content is 0.8% to 0.9%, the specific heat is 0.5-0.78 cal/g · °C, which is a desirable heat preservation material. When each kilogram of paraffin melted upon being heating is cooled and solidified, the released melting heat is 39 cal on the average, and it maintains the temperature of 40°C-48°C in 70 min to 90 min, which has the features of high heat capacity, small thermal conductivity, almost no heat convection, and desirable heat preservation. In 1970s, reports from many European laboratories approved that paraffin has significant desirable effects on the skin care.

The nutritive substances easily absorbed by the skin include Vitamin E, collagen, or plant essential oils such as lavender, peach, and tea.

The step of cleaning the skin under treatment includes using a body scrub containing particulate matters to scrub on the surface of the skin under treatment, and using a body lotion to massage the surface of the skin under treatment. The former process can remove dead skins and horny layer, and the latter process can accelerate the blood circulation, which is helpful for the skin to absorb the nutritive substances.

The step of performing the sealed heat-insulation and heat-preservation process on the wax mask is to use an airtight heat-insulating material to wrap the wax mask, so as to prevent the heat energy stored in the paraffin from being dispersed outside, but make the heat energy be delivered to the deep tissues via the surface of the skin, so as to promote sweat glands to discharge sweats and metabolism effect of skin tissues, to expand the capillary vessel, and to accelerate the blood circulation.

The duration for the step of performing the sealed heat-insulation and heat-preservation process on the wax mask is 10 min to 30 min, and preferably 15 min to 20 min.

The skin under treatment is hand skin, foot skin, leg skin, arm skin, breast skin, or skin from head to foot.

As for the skin-treatment tool using wax of the present invention, the above technical problem is solved as follows.

The skin-treatment tool using wax includes a heating device and containers filled with a skin-treatment wax and disposed in the heating device.

The skin-treatment tool using wax is characterized in that, the heating device is a heating device used for preheating the skin-treatment wax filled in the containers disposed in the heating device to melt the wax into a liquid wax capable of being sprayed; and the containers filled with the skin-treatment wax are at least one container with nozzles disposed in the heating device.

In this manner, it does not require to use the brush or blade to coat the liquid wax on the surface of the skin under treatment, or to immerge the surface of the skin under treatment within the bath filled with the liquid wax. Therefore, the cross infection of the skin bacteria and virus is not generated, and the liquid wax in the containers with the nozzles is not solidified during the spraying process away from the heating device, and thus can be operated continuously.

As for the skin-treatment tool using wax of the present invention, the above technical problem can be preferably solved as follows.

Each of the containers with nozzles is formed by a triggering or pressing nozzle and a container body connected together in a manner of screwing.

The heating device includes: a case, having a front case and a back case; a heating chamber, disposed within the case; and a heating element, disposed on a side wall or a bottom wall of the heating chamber, the heating element converts an energy supplied to the heating device into a heat energy, and preheats the skin-treatment wax in the containers with nozzles disposed in the heating chamber to melt the wax into the liquid wax capable of being sprayed.

As for the skin-treatment tool using wax of the present invention, the above technical problem can be further solved as follows.

Each of the containers with nozzles is a container made of a transparent material, so that it is convenient for the user to observe the melting state of the skin-treatment wax in the containers.

Each of the containers with nozzles is a one-time product made of polythene or polypropylene.

The front case of the heating device is in an open configuration, and has a front cover that is transparent and can be turned over or has a transparent window. That is, the front cover can be totally made of a transparent material, and merely a transparent window for observation made by a transparent material is formed at a position corresponding to the container body with the nozzle, such that it is convenient for the user to put in and take out the containers with nozzles, and to observe the melting state of the skin-treatment wax placed in the heating chamber.

The heating device has at least two front covers, only one of them is randomly opened for putting in or taking out the containers with nozzles, so as to reduce the dispersion of the energy in the heating chamber.

The number of the containers with nozzles filled with the skin-treatment wax disposed in the heating chamber of the heating device, i.e., the total amount of the liquid wax capable of being sprayed, corresponds to the total area of the surface of the skin under treatment, and the total amount of the liquid wax in the heating chamber is at least the dosage required by completing a whole skin treatment process using wax.

The heating chamber of the heating device is formed by at least two heating chamber units arranged side by side. Each heating chamber unit has at least one container with nozzles filled with the skin-treatment wax, so that a plurality of containers with the nozzles is disposed in order, which is convenient for being used.

The heating chamber units of the heating device are fabricated through performing pressure casting on aluminum alloy, so as to improve the heat energy conductance provided by the heating element to the heating device, and thus more effectively preheating the skin-treatment wax in the containers with nozzles in the heating chamber.

The heating device is a heating device employing an electric heating component.

The heating element of the heating device employing the electric heating component is a resistor electric heating component using a metal material, such as a resistor heating wire, electric heating tube, and electric heating plate.

The heating element of the heating device is a far infrared electric heating component with far infrared radiation substance being coated on the surface of the metal material, such as far infrared heating wire, far infrared heating tube, and far infrared heating plate.

The heating element of the heating device employing the electric heating component is a resistor electric heating component using a non-metal material, such as a positive temperature coefficient electric heating component.

The heating device can also be a heating device employing a chemical energy-storage heating element using activated carbon fiber or activated carbon as a carrier. The heat supply temperature of the chemical energy-storage heating element is kept at 100°C to 120°C persistently for tens of minutes after the water injection, so as to preheat the skin-treatment wax in the containers with nozzles to melt the wax into a liquid wax capable of being sprayed, and thus, people can take along during a wild tour or in places where the external AC power source is unavailable.

The heating device employing the electric heating component further includes a power line, a power plug, a power switch, and an electrical control member connected to the external AC power source.

The heating device employing the electric heating component is a temperature control heating device, and the electrical control member includes a temperature controller. The temperature range of the heating chamber is 45°C to 85°C, so as to cater to the temperature characteristics of different kinds of skin-treatment waxes, and thus the heated skin-treatment wax maintains the liquid state capable of being sprayed.

The skin-treatment method using wax and the tool thereof of the present invention is a novel skin-treatment method and a tool thereof proposed based on using the heating device to preheat the skin-treatment wax to melt the wax into a liquid wax capable of being sprayed, such that the skin tissues sufficiently enjoyed the heating effect for a long time duration, so as to achieve preferred treatment function. It is simple, easy, and safe, especially in the aspect that the liquid wax is sprayed on the surface of the skin under treatment, without using the brush or blade to coat the liquid wax on the surface of the skin under treatment, or immerging the surface of the skin under treatment in the bath filled with the liquid wax. The cross infection of the skin bacteria and virus is not generated, the sanitary requirement is satisfied, and the liquid wax is not solidified during the spraying process away from the heating device, and thus can be operated continuously. The skin-treatment tool using the wax of the present invention has a compact structure and is convenient for being used, which has been widely used in beauty parlors, houses, apartments, hotel rooms, lounges, and other places capable of performing the skin treatments using wax on the spot. As for the skin-treatment tool employing the chemical energy-storage heating element, people can take along in the wild tour or in places where the external AC power source is unavailable.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### BRIEF DESCRIPTION OF THE DRAWINGS

The present invention will become more fully understood from the detailed description given herein below for illustration only, which thus is not limitative of the present invention, and wherein:

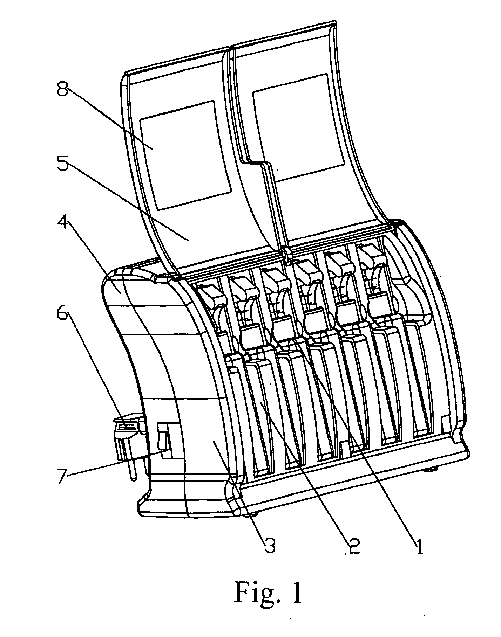

FIG 1 is a schematic perspective view of a configuration of a heating device for a treatment tool according to an embodiment of the present invention.

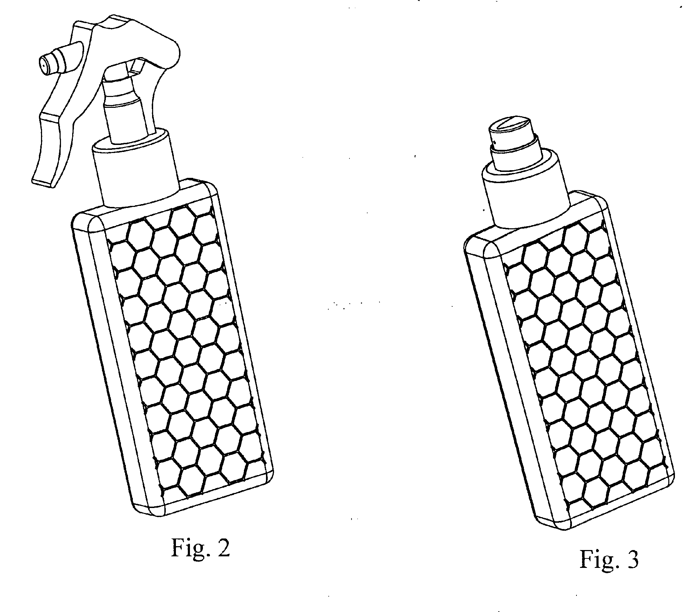

FIG 2 is a schematic perspective view of a container with a triggering nozzle for the treatment tool according to an embodiment of the present invention.
FIG 3 is a schematic perspective view of a container with a pressing nozzle for the treatment tool according to an embodiment of the present invention.

### DETAILED DESCRIPTION OF THE INVENTION

A hand-skin-treatment method using wax and a tool thereof are provided.

The hand-skin-treatment method using wax sequentially includes the following steps.
(1) The medical paraffin filled in the containers with nozzles in the heating device with a temperature range of 45°C-85°C is preheated to be melted into a liquid wax capable of being sprayed, and the kinematic viscosity is 3.2-4.2 centistokes (98.9°C).
(2) The hand skin under treatment is cleaned, including using the body scrub containing particulate matters to scrub on the surface of the hand skin under treatment, and using the body lotion to massage the surface of the hand skin under treatment.
(3) The liquid wax filled in the containers with nozzles in the heating device is uniformly sprayed on the surface of the hand skin under treatment to form a layer of wax, and then the liquid wax is repeatedly sprayed to form at least two layers of wax, so as to form the wax mask with a thickness of 1.2 mm-1.6 mm.
(4) The airtight heat insulating plastic film is firstly used to wrap the wax mask, and then a towel glove is covered thereon, so as to perform the sealed heat-insulation and heat-preservation process on the wax mask for a time duration of 18 minutes.
(5) The solidified and thoroughly-dried wax mask is removed.

Preferably, the treatment is performed once a week, and the distinct effect will be achieved after ten times of continuous treatments.

The skin-treatment tool using a wax of this embodiment includes a heating device and containers filled with the medical paraffin.

The containers filled with the medical paraffin are six containers 1 with the triggering nozzle made of transparent polypropylene material and they are one-time products. The triggering nozzle is connected to the rectangular container body in the manner of screwing, and the capacity of each container is 100 ml.

The heating device includes: a case, having a front case 3 and a back case 4; a heating chamber 2, disposed within the case; and a heating element, disposed on a side wall or a bottom wall of the heating chamber 2. The heating chamber 2 is formed by six heating chamber units arranged side by side, and the heating chamber units are fabricated through performing pressure casting on aluminum alloy. Each heating chamber unit has a container with the triggering nozzle 1, the front case 3 in the opening configuration, and having two front covers 5 that is turned over and has a transparent window 8. Only one of the two covers 5 is randomly opened for putting in and taking out the container with the triggering nozzle 1.

The heating device further includes a power line, a power plug 6, a power switch 7, and an electrical control member connected to the external AC power source.

The electrical control member includes a temperature controller. The temperature range of the heating chamber is 45°C to 85°C.

Firstly, according to the temperature characteristics of the employed medical paraffin, the temperature controller is adjusted to an appropriate temperature control range. Then, the power switch 7 is turned on, the heating element of the electric heating tube uses a heat sink fin to preheat the medical paraffin filled in the container with the triggering nozzle 1 disposed in the heating chamber 2 to melt the medical paraffin into the liquid wax capable of being sprayed, and the liquid wax is maintained at a set temperature range for later use.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

## Claims

1. A skin-treatment method using wax, sequentially comprising: preheating a skin-treatment wax to melt the wax into a liquid wax, cleaning skin under treatment, forming a wax mask on the surface of the skin under treatment, performing a sealed heat-insulation and heat-preservation process on the wax mask, and removing the solidified and thoroughly-dried wax mask, wherein:
the step of preheating the skin-treatment wax to melt the wax into a liquid wax is to preheat the skin-treatment wax filled in containers with nozzles in a heating device to melt the wax into the liquid wax capable of being sprayed;
the step of forming the wax mask on the surface of the skin under treatment is to spray the liquid wax filled in the containers with nozzles in the heating device onto the surface of the skin under treatment to form the wax mask.

2. The skin-treatment method using wax as claimed in claim 1, wherein:
the step of spraying the liquid wax on the surface of the skin under treatment to form the wax mask is to uniformly spray the liquid wax to form a layer of wax and then to spray the liquid wax repeatedly to form several layers of wax, so as to form the wax mask.

3. The skin-treatment method using wax as claimed in claim 1 or 2, wherein:
the liquid wax used for spraying has a kinemetic viscosity cSt of 3.2 centistokes to 4.2 centistokes (98.9°C), or has Saybolt Universal Second (SUS) of 37 seconds to 40 seconds (98.9°C).

4. A skin-treatment tool using wax, comprising a heating device and containers filled with the skin-treatment wax and disposed in the heating device, wherein:
the heating device is a heating device used for preheating the skin-treatment wax placed therein to melt the wax into a liquid wax capable of being sprayed;
the containers filled with the skin-treatment wax are at least one container with nozzles disposed in the heating device.

5. The skin-treatment tool using wax as claimed in claim 4, wherein:
each of the containers with nozzles is formed by a triggering or pressing nozzle and a container body connected together by means of screwing.

6. The skin-treatment tool using wax as claimed in claim 4 or 5, wherein:
each of the containers with nozzles is a container made of a transparent material.

7. The skin-treatment tool using wax as claimed in claim 4, wherein:
the heating device comprises: a case, having a front case and a back case; a heating chamber, disposed within the case; and a heating element, disposed on a side wall or a bottom wall of the heating chamber, the heating element converts an energy supplied to the heating device into a heat energy for preheating the skin-treatment wax within the containers with nozzles disposed in the heating chamber to melt the wax into the liquid wax capable for being sprayed.

8. The skin-treatment tool using wax as claimed in claim 7, wherein:
the number of the containers with the nozzle filled with the skin-treatment wax in the heating chamber of the heating device, i.e., the total amount of the filled liquid wax capable of being sprayed, corresponds to a total area of the skin under treatment; and the total amount of the liquid wax, capable of being sprayed, in the heating chamber is at least a dosage required by completing a whole skin-treatment process using wax.

9. The skin-treatment tool using wax as claimed in claim 8, wherein:
the heating device is a heating device employing an electric heating component, or a heating device employing a chemical energy-storage heating element using activated carbon fiber or activated carbon as a carrier.

10. The skin-treatment tool using wax as claimed in claim 9, wherein:
the heating device employing the electric heating component is a temperature control heating device, a temperature range of the heating chamber is 45°C to 85°C, so as to cater to temperature characteristics of different kinds of skin-treatment waxes, such that the heated skin-treatment wax maintains the liquid state capable of being sprayed.
